# EUROPEAN PATENT SPECIFICATION

(11) **EP 0 421 562 B1**
(45) Date of publication and mention of the grant of the patent: **07.05.1997**
(21) Application number: 90250257.4
(22) Date of filing: 05.10.1990
(51) Int. Cl.: A61K 31/557

(54) **Use of prostacyclin analogs against metastases**
Verwendung von Prostacyclin-Analoga gegen Metastasen
Utilisation d'analogues de la prostacycline contre les métastases

(30) Priority: 05.10.1989 DE 3933523
(43) Date of publication of application: 10.04.1991
(73) Proprietor: SCHERING AKTIENGESELLSCHAFT, 13342 Berlin (DE)
(72) Inventor: Schneider, Martin, W-1000 Berlin 15 (DE); Schillinger, Ekkehard, W-1000 Berlin 28 (DE)

(56) References cited:
- EP-A- 0 099 538
- EP-A- 0 366 216
- WO-A-87/05294
- ADVANCES IN PROSTAGLANDIN, THROMBOXANE, AND LEUKOTRIENE RESEARCH, vol. 21B, Prostaglandins and Related Compounds, Seventh International Conference, Florence, Italy, edited by B. Samuelsson et al., 1990, pages 913-916, Raven Press, Ltd, New York, US; t. GIRALDI et al.: "Antimetastatic action of stable prostacylin analogs in mice"
- ADVANCES IN PROSTAGLANDIN, THROMBOXANE, AND LEUKOTRIENE RESEARCH, vol. 21B, Prostaglandins and Related Compounds, Seventh International Conference, Florence, Italy, edited by B. Samuelsson et al., 1990, pages 901-908, Raven Press, Ltd, New York, US; M.R. SCHNEIDER et al.: "Effects of prostacyclin analogues in in vivo tumor models"

## Description

### Background of the Invention

This invention relates to a new agent with antimetastatic action.

After application of prostacyclin (PGI₂) in high dosage (10 mg/kg) shortly before intravenous injection of tumor cells of B16 mouse melanoma, K.V. Honn et al. [Science 212, 1270 (1981)] observed a reduction of the number of lung metastases. This action is ascribed to an inhibition of the tumor cell adhesion and aggregation. But for an application the half-life is too short and the dose of PGI₂ is too high. Therefore, with the more stable prostacylin analog, Iloprost, a pharmaceutical application would be more conceivable.

V. Constantini et al. [Cancer Chemother. Pharmacol. 22, 289 (1988) compared the antimetastatic action of PGI₂ (5 and 10 mg/kg) and Iloprost (0.1 and 0.2 mg/kg) on the formation of lung metastases of the melanoma cell line BLG after intravenous injection of the tumor cells in mice. The action was a function of the number of applied tumor cells and the periods between substance and tumor cell application. In various test arrangements, Iloprost proved to be clearly superior to PGI₂ from the dose, the duration of action and the achieved inhibition action.

Piccini et al. (1988) obtained comparable data on the Lewis lung carcinoma of the mouse by use of the same dosages and similar test arrangements. In a one-time dose of 0.2 mg/kg, 1 hour before injection of the tumor cells, Iloprost reduced the number of lung metastases by more than 90%.

But test arrangements such as intravenous injection of tumor cells or one-time substance administration clinically have little relevance.

Another test described by Piccini et al. is more meaningful. In a standard model for establishing an antimetastatic potency, namely i.m. implantation of a primary tumor (Lewis lung carcinoma) in the leg and subsequent surgical removal of the tumor (leg amputation), Iloprost (0.2 mg/kg, 1.5 hours before tumor removal) reduced the number of lung metastases by about 50%. But the survival time was only insignificantly lengthened.

### Summary of the Invention

It has now been surprisingly found that Eptaloprost, Cicaprost in contrast with the action known so far of other prostacyclins, act selectively antimetastatically, since no action on the growth of the primary tumor can be detected.

The invention thus relates to the use of Eptaloprost, Cicaprost, their addition salts with physiologically compatible bases, their clathrates with cyclodextrins, in combination with the usual auxiliary agents and vehicles for the manufacture of antimetastatically acting medicaments.

Eptaloprost [(5E)-(16S)-13,14-didehydro-1a,1b-dihomo-16,20-dimethyl-3-oxa-18,18,19,19-tetradehydro-6a-carbaprostaglandin-I₂] and its beta-cyclodextrin clathrate can be produced, for example, according to the following formulation:

17.2 ml of a 50% sodium hydroxide solution and 337 mg of tetrabutyl ammonium hydrogen sulfate are added to a mixture of 6.9 g of 2-[(E)-(1S,5S,6S,7R)-7-(dimethyl-tert-butylsilyloxy)-6-[(3S,4S)-3-(dimethyl-tert- butylsilyloxy)-4-methyl-nona-1,6-diinyl]bicyclo[3.3.0]octan-3-ylidene]-ethan-1-ol (W. Skuballa, E. Schillinger, C.-S. Stuerzebecher, M. Vorbruggen, J. Medicinal Chemistry 29, 313 (1986); described here as compound 15a) and 11.5 g of trimethyl-ortho-4-bromobutyrate and stirred for 16 hours at 22°C under argon. Then with ice water cooling it is diluted with 20 ml of water and acidified with 10% citric acid solution to pH 5. It is extracted three times with 300 ml of ether each, the organic phase is washed once with 200 ml of brine, dried on magnesium sulfate and concentrated by evaporation in a vacuum. After chromatography of the residue on silica gel with hexane/ether (8+2), 7.6 g of (5E)-(16S)-13,14-didehydro-1a,1b-dihomo-16,20-dimethyl-3-oxa-18,18,19,19-tetra-dehydro-6a-carbaprostaglandin-I₂-methylester-11,15-bis-(dimethyl-tert-butyl-ether) is obtained as a colorless oil.

IR (CHCl₃): 2953, 2925, 2859, 2230, 1730, 1250, 838 cm⁻¹.

For silyl ether cleavage, 7.25 g of the above-described bis-silyl ether is stirred for 48 hours at 24°C with 600 ml of a mixture of acetic acid/water/tetrahydrofuran (65+35+10). Then it is concentrated by evaporation in a vacuum and the residue is chromatographed on silica gel. With ethyl acetate/hexane (3+2), 3.9 g of (5E)-(16S)-13,14-didehydro-1a,1b-dihomo-16,20-dimethyl-3-oxa-18,18,19,19-tetrahydro-6a-carbaprostaglandin-I₂-methylester is obtained as a colorless oil.

IR (CHCl₃): 3400 (broad), 2935, 2865, 2230, 1735 cm⁻¹.

For saponification, a solution of 3.66 g of the above-produced methylester is stirred in 35 ml of methanol with 35 ml of an 0.5 molar sodium hydroxide solution for 30 minutes at 24°C under argon. Then it is diluted with 20 ml of water, acidified with a 20% citric acid to pH 2, extracted 4 times with 100 ml of methylene chloride each, the organic phase is washed once with 50 ml of brine, dried on sodium sulfate and concentrated by evaporation in a vacuum. The residue is chromatographed with ethyl acetate on silica gel. Thus 3.4 g of the title compound is obtained as a colorless oil.

IR (CHCl₃): 3400 (broad), 2962, 2940, 2865, 2230, 1722 cm⁻¹.

Beta-cyclodextrin clathrate of (5E)-(16S)-13,14-didehydro-1a,1b-dihomo-16,20-dimethyl-3-oxa-18,18,19,19-tetra-dehydro-6a-carbaprostaglandin-I₂.

41.75 g of beta-cyclodextrin is dissolved in 298 ml of water at 80°C and a solution of 1.5 g of (5E)-(16S)-13,14-didehydro-1a,1b-dihomo-16,20-dimethyl-3-oxa-18,18,19,19-tetradehydro-6a-carbaprostaglandin-I₂ in 24 ml of ethanol is instilled in 15 minutes. It is stirred for 4 hours at 60°C and then allowed to cool overnight with stirring. The precipitated solid is suctioned off, washed with 50 ml of a mixture of water-ethanol (1:1) and dried for 24 hours at 0.1 torr and 25°C on phosphorus pentoxide. 38 g of free-flowing crystals of the betacyclodextrin clathrate of the above-mentioned carbacyclin analog is obtained. The content of carbacyclin analogs in the clathrate is determined by titration and amounts to 3.3%.

Cicaprost and its beta-cyclodextrin clathrate can be produced according to the processes described in EP-PS 119,949 (CA 1,251,200) and in international laid-open specification WO 87/05294 (U.S. 4,886,788).

With intravenous infusion of Eptaloprost, Cicaprost, amounts of prostacyclin or prostacyclin analog, for example, of about 1-50 micrograms per patient per day, preferably 12.5 µg/patient/day, are necessary. In oral application, about 125-350 micrograms per patient per day is used.

For the oral application a dose unit contains 25-50 micrograms of prostacyclin or prostacyclin analog, in sustained release formulations 25-250 micrograms, as tablet, coated tablet, capsule, pill, suspension or solution, which can be produced in the usual way with additives and vehicles usual in galenicals.

Where the prostacyclins are used along with mixtures of their clathrates and salts, the ratio of one given active agent to another is not important, so long as the biologically equivalent amount of overall active agent is within the quoted ranges.

For the preferred oral application especially tablets, coated tablets, capsules, pills, suspensions or solutions are suitable, which are mixed in the usual way with additives and vehicles usual in galenicals.

β-Cyclodextrin is preferable as a cyclodextrin for combination with the prostacyclins.

The invention relates to the use of Eptaloprost, Cicaprost for the production of a pharmaceutical agent with antimetastatic action. Generally, all malignant human tumors (except leukema-based cancers) produce metastacization, and the invention is applicable thereto.

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the foregoing and in the following examples, all temperatures are set forth uncorrected in degrees Celsius; and, unless otherwise indicated, all parts and percentages are by weight.

The entire disclosures of all applications, patents, and publications, cited above and below, and of corresponding German Application P 39 33 523.2, filed October 5, 1989, are hereby incorporated by refrence.

### EXAMPLE

The cell line R 3327 MAT-Lu is used in the following tests. This cell line is discussed in the review article of J.T. Isaacs (Current Concepts and Approaches to the Study of Prostate Cancer 513-576, 1987, Alan R. Liss, Inc., Animal Model Systems), on page 533. The researchers and laboratory indicated as first isolating this MAT-Lu subline were D.W. Lanzan, W.D.W. Heston, D. Kadmon and W.R. Fair (Division of Urology, Dept. of Surgery, Washington University School of Medicine, St. Louis). Moreover, in the above article, animal strains are described in which this subline develops, and detailed growth characteristics of this cell line are indicated. D.W. Lazan et al. describe in Cancer Research 42,1390 (1982), how an individual cell suspension of R 3327 MAT-Lu tumor cells in prepared with a freshly excised side tumor from an individual rat.

The R 3327 MAT-Lu, obtained by Dr. Isaacs of John Hopkins University, is fast-growing, endocrine-independent and exhibits no measurable steroid receptors. The metastasizing rate is 100%. The survival time of animals after s.c. implantation is about 35 days. An arrangement with s.c. implantation without removal of the primary tumor was selected to detect an antimetastatic action of prostacyclin derivatives or of the combination products of TXA₂ antagonist and prostacyclins.

1. The tumor is removed under aseptic conditions from the donor animal about 4 weeks after implantation. In MEM 199 (minimum essential medium, standardized medium of Gibco company, UK) the tumor is freed of necrotic portions and cut in pieces about 2 mm edge length. One tumor piece each is implanted subcutaneously in adult, male Cop rats (OLAC, England). The animals are randomized to 4 groups of 10 animals each. Already on the previous day, Alzet minipumps were implanted s.c. in the animals. Group 1: solvent; group 2: Iloprost (300 ng/kg/min); group 3: Eptaloprost (100 ng/kg/min). On day 16 the pumps are replaced.

The tumors are measured once a week with a sliding caliper. The tumor surface is calculated as the product of the greatest diameter and of the diameter perpendicular to it. At the end of the test the animals are killed by decapitation. The level of active ingredients in the blood is determined. The lungs are dissected, fixed in Bouin solution for 24 hours and then preserved in alcohol. The number of lung metastases is determined by counting. This is made easier because metastases cannot be stained by Bouin. Further, a classification into "large" and "small" metastases is performed.

### 2. Action of Iloprost and Eptaloprost on R 3327 MAT-LU PC

In the selected test arrangement (s.c. implantation of the tumor, no removal of the primary tumor), the requirements placed on the antimetastatic action are classified as very high, since the primary tumor continuously produces metastases.

At the end of the test the body weight was not significantly reduced under therapy with prostacyclins

| | Body Weight (g) | Tumor Surface (mm²) |
|---|---|---|
| Control | 315 ± 28 | 1966 ± 791 |
| Iloprost | 282 ± 43 | 1658 ± 485 |
| Eptaloprost | 281 ± 43 | 1928 ± 691 |

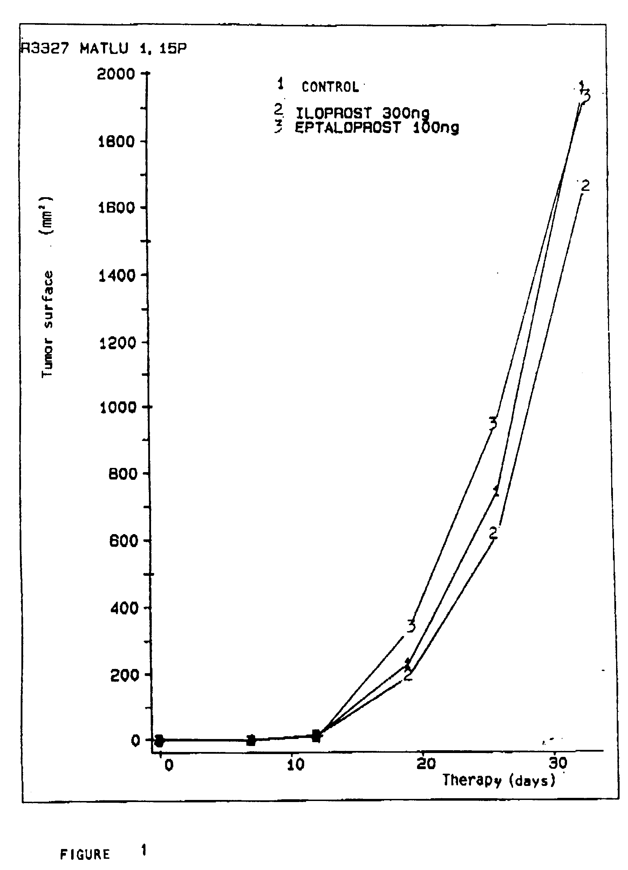

The tumor growth took place very progressively starting from about day 12 after implantation (fig. 1). A specific inhibitor of metastasizing should have no effect on the growth of the primary tumor. With Iloprost and Eptaloprost there is also no difference in the growth of the primary tumor in comparison with the control.

The evaluation of the lung metastases of this PC metastasizing only in the lung provides solely information on the antimetastatic potential. The located metastases vary in their size from less than 1 mm in diameter to about 3 mm. A diversion into "small" and "large" metastases can be made without the occurrence of many borderline cases.

With Iloprost no reduction of the total number of lung metastases can be detected in this model. However, Eptaloprost results in significant and marked inhibition of the total number of metastases (fig. 2). In a division into "large" and "small" metastases for Iloprost there is a trend to the reduction of the number of "large" metastases (fig. 3), while the number of "small" metastases in comparison with the control has a tendency to increase (fig. 4). Also the marked effect on the metastasis number with Eptaloprost is more strongly pronounced with "large" metastases than with "small" metastases.

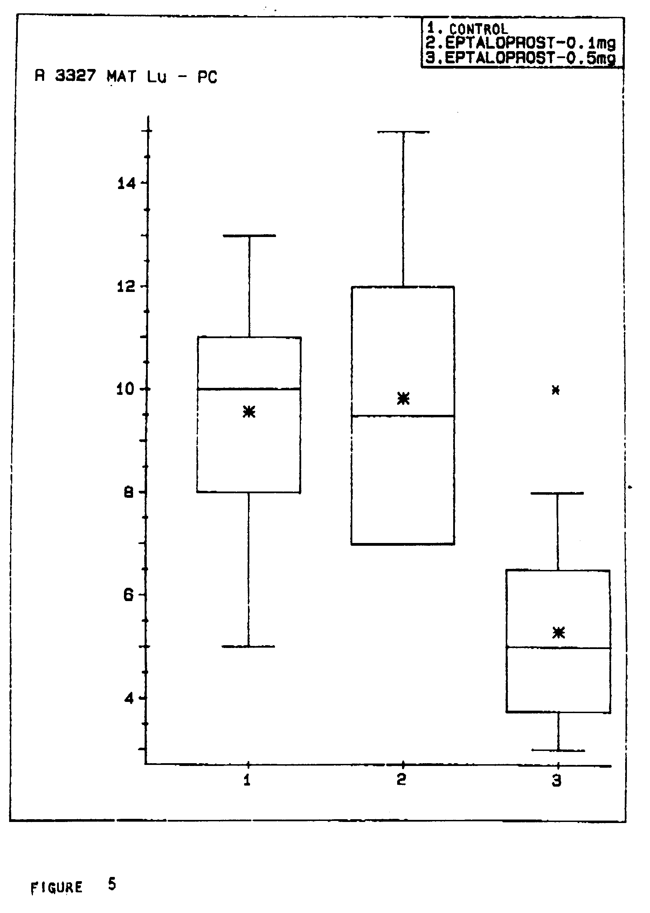

3. In a second experiment with the same test arrangement as under 2., Eptaloprost is applied in doses of 0.1 to 0.5 mg/kg daily orally from the day of tumor implantation to the end of the test. Again there is no action on the primary tumor. While in the 0.1 mg/kg dose, no effect on the number of lung metastases can be detected, Eptaloprost in the higher dose of 0.5 mg/kg of body weight results in a significant (p less than 0.05, Dunnett test) reduction of the metastasis number (fig. 5).

Following are the meanings of Figures 1-5:
Fig. 1: Action of Iloprost and Eptaloprost on the growth of R 3327 MAT-Lu (primary tumor).

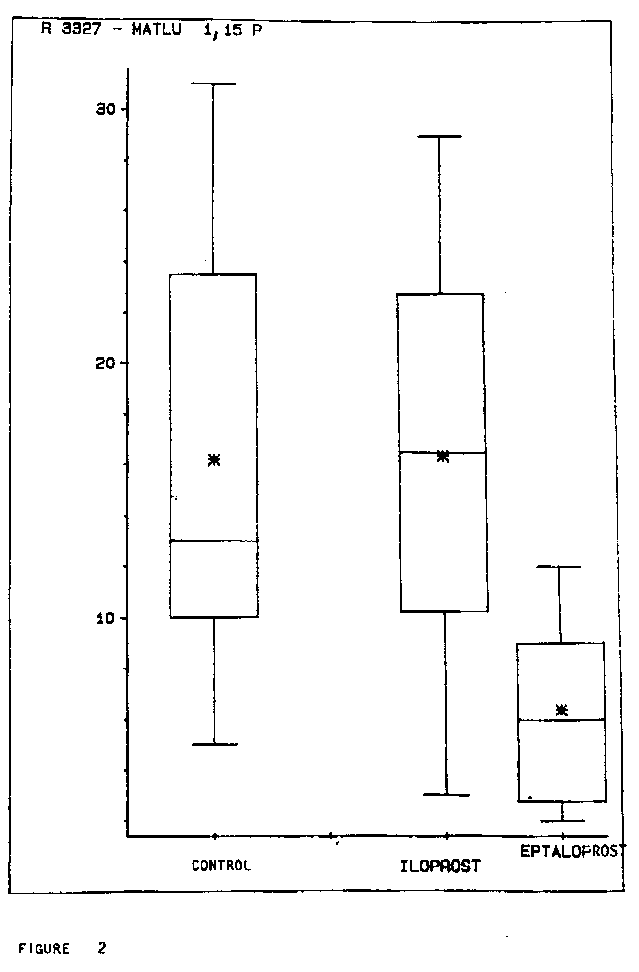

Fig. 2: Action of Iloprost and Eptaloprost on the total number of lung metastases.

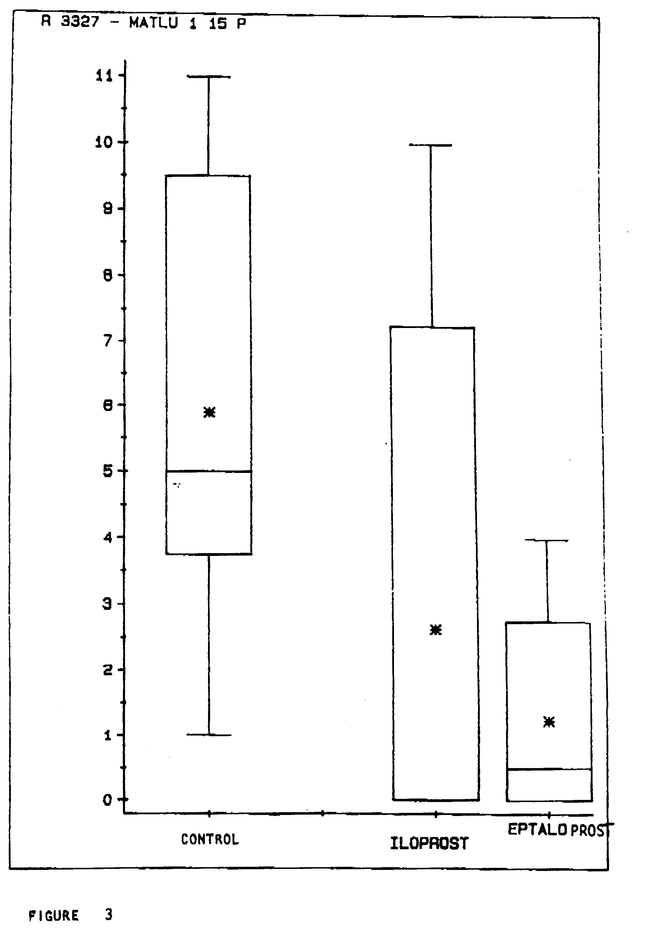

Fig. 3: Action of Iloprost and Eptaloprost on the number of "large" lung metastases.

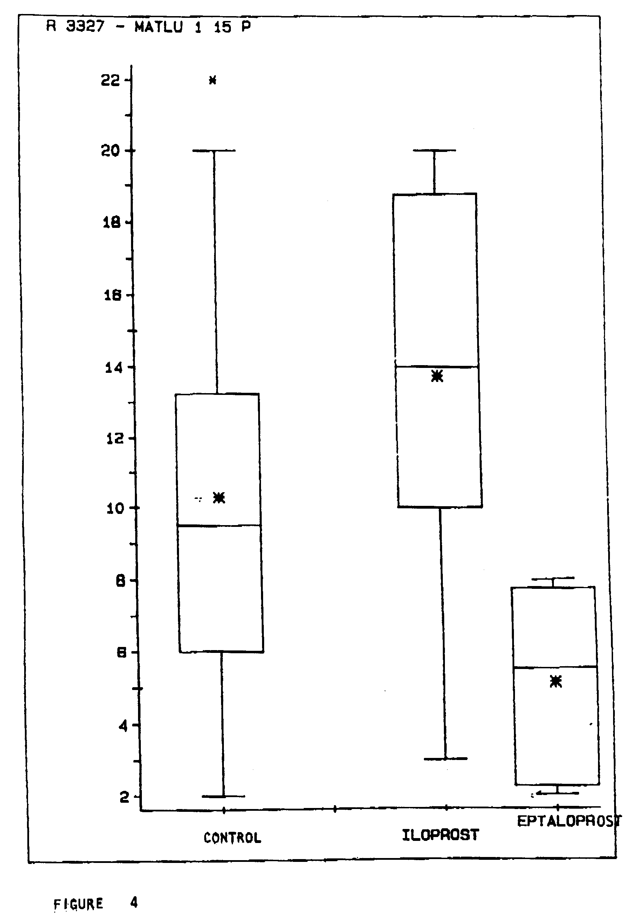

Fig. 4: Action of Iloprost and Eptaloprost on the number of "small" lung metastases.
Fig. 5: Action of Eptaloprost in various dose amounts on the number of lung metastases.

## Claims

1. The use of Eptaloprost, Cicaprost, an addition salt of Eptaloprost or Cicaprost with a physiologically compatible base, a cyclodextrin clathrate, or a mixture thereof for the manufacture of a pharmaceutical composition for the treatment of metastatic activity in a host.

2. The use of Eptaloprost as claimed in claim 1.

3. The use of Eptaloprost cyclodextrin clathrate as claimed in claim 1.

4. The use of Cicaprost as claimed in claim 1.

5. The use of Cicaprost cyclodextrin clathrate as claimed in claim 1.

6. The use of Eptaloprost, Cicaprost, an addition salt of Eptaloprost or Cicaprost with a physiologically compatible base, a cyclodextrin clathrate, or a mixture thereof for the manufacture of a pharmaceutical composition for the treatment of malignant tumors in a human host wherein the mentioned compounds or mixtures are administered in an effective amount.

## Patentansprüche

1. Verwendung von Eptaloprost, Cicaprost, eines Additionssalzes von Eptaloprost oder Cicaprost mit einer physiologisch verträglichen Base, eines Cyclodextrinclathrats oder eines Gemisches hievon für die Herstellung einer pharmazeutischen Zusammensetzung zur Behandlung von metastatischer Aktivität in einem Wirt.

2. Verwendung von Eptaloprost nach Anspruch 1.

3. Verwendung von Eptaloprostcyclodextrinclathrat nach Anspruch 1.

4. Verwendung von Cicaprost nach Anspruch 1.

5. Verwendung von Cicaprostcyclodextrinclathrat nach Anspruch 1.

6. Verwendung von Eptaloprost, Cicaprost, eines Additionssalzes von Eptaloprost oder Cicaprost mit einer physiologisch verträglichen Base, eines Cyclodextrinclathrats oder eines Gemisches hievon für die Herstellung einer pharmazeutischen Zusammensetzung zur Behandlung von malignen Tumoren in einem menschlichen Wirt, wobei die erwähnten Verbindungen oder Gemische in einer wirksamen Menge verabreicht werden.

## Revendications

1. Utilisation de''eptaloprost, de cicaprost, d'un sel d'addition d'eptaloprost ou de cicaprost avec une base physiologiquement compatible, d'un clathrate de cyclodextrine, ou d'un mélange de ces composés pour la préparation d'une composition pharmaceutique pour le traitement de l'activité de formation de métastases dans un hôte.

2. Utilisation de l'eptaloprost revendiquée dans la revendication 1.

3. Utilisation du clathrate de cyclodextrine de l'eptaloprost selon la revendication 1.

4. Utilisation du cicaprost selon la revendication 1.

5. Utilisation du clathrate de cyclodextrine de cicaprost selon la revendication 1.

6. Utilisation de l'eptaloprost, du cicaprost, d'un sel d'addition d'eptaloprost ou de cicaprost avec une base physiologiquement compatible, d'un clathrate de cyclodextrine, ou d'un de leurs mélanges pour la préparation d'une composition pharmaceutique pour le traitement de tumeurs malignes dans un hôte humain en administrant les composés ou les mélanges cités en une quantité efficace.
